(19) **Europäisches Patentamt**

**European Patent Office**

**Office européen des brevets**

(11) **EP 1 244 058 A1**

(12) **EUROPÄISCHE PATENTANMELDUNG**

(43) Veröffentlichungstag:
   **25.09.2002 Patentblatt 2002/39**

(51) Int Cl.⁷: $G06T\ 11/00$

(21) Anmeldenummer: **01107128.9**

(22) Anmeldetag: **22.03.2001**

(84) Benannte Vertragsstaaten:
   **AT BE CH CY DE DK ES FI FR GB GR IE IT LI LU
   MC NL PT SE TR**
   Benannte Erstreckungsstaaten:
   **AL LT LV MK RO SI**

(71) Anmelder: **VAMP Verfahren und Apparate der
   Medizinischen Physik GmbH
   91096 Möhrendorf (DE)**

(72) Erfinder:
   • **Kachelriess, Marc, Dr.
     90409 Nürnberg (DE)**
   • **Kalender, Willi A., Dr.
     91096 Kleinseebach (DE)**

(54) **Computertomograph mit merkmalsgewichteter Bild-zu-Volumen Interpolation in Echtzeit**

(57)   Die vorliegende Erfindung beschreibt ein Verfahren, das zweidimensionale, beliebig im Raum orientierte primäre Bilddaten nach Merkmalen, die den jeweiligen Bildern oder bestimmten Bildbereichen zugeordnet werden, in Echtzeit zu gewichten und in einen regelmäßigen Volumendatensatz umzuinterpolieren. Das Wichtungsverfahren ermöglicht es, beliebige Kombinationen (z.B. Überblendungen) unterschiedlicher, ortsaufgelöster Informationen manuell oder automatisch darzustellen und zu variieren.

Printed by Jouve, 75001 PARIS (FR)

## Beschreibung

Die moderne Röntgen-Computer-Tomographie (CT) hat durch neue Scanverfahren und durch Verbesserungen der Technologie ein breites Anwendungsspektrum erreicht. Die Spiral-CT, also kontinuierliche Datenakquisition bei gleichzeitigem Patientenvorschub, ermöglicht sehr schnelle Volumenaufnahmen [W.A. Kalender, W. Seissler, E. Klotz, P. Vock. Spiral volumetric CT with single-breathhold technique, continuous transport, and continuous scanner rotation. Radiology 176 (1):181-183, 1990][W.A. Kalender. Computertomographie. MCD Publicis, Erlangen 2000]. Bei so genannten Mehrschicht-Spiral-CT (MSCT) Scannern werden zur Zeit bis zu 4 Schichten pro Umlauf gemessen; die Rekonstruktion der Daten erfolgt unter der Annahme, diese 4 Schichten seien parallel zueinander [T. Fuchs, M. Kachelrieß, W.A. Kalender. Technical advances in multi-slice spiral CT. European Journal of Radiology 36(2):69-73, November 2000]. Um die damit möglichen Volumenaufnahmen weiter zu beschleunigen, werden Scanner mit einer noch höheren Anzahl simultan gemessener Schichten entwickelt. Die Annahme paralleler Schichten ist dann nicht mehr gerechtfertigt, man spricht von Kegelstrahl- oder Cone-Beam-CT (CBCT) [W.A. Kalender. Computertomographie. MCD Publicis, Erlangen 2000]. Die gleichzeitige Erfassung von bis zu 16 Schichten oder mehr ist für die nahe Zukunft zu erwarten. Die CBCT erfordert den Einsatz neuer Rekonstruktionsverfahren, welche die Kegelstrahlgeometrie des Scanners explizit berücksichtigen. Moderne Kegelstrahlrekonstruktionsalgorithmen teilen sich in zwei Bereiche auf: die exakten und die approximativen Algorithmen [Henrik Turbell. Cone-beam reconstruction using filtered backprojection. PhD Thesis, Linköpings Universitet, 2001]. Exakte Algorithmen basieren auf einer mathematisch exakten Inversion der gemessenen Daten; die auftretenden Diskretisierungsprobleme, die damit verbundenen Artefakte und die sehr hohen Rekonstruktionszeiten sind jedoch unakzeptabel für einen Einsatz in der klinischen CT. Approximative Algorithmen hingegen sind effizient in der Rechenzeit, weisen aber häufig zu starke Bildartefakte auf. Seit kurzem steht jedoch ein neuartiger, nahezu exakter approximativer Kegelstrahlrekonstruktionsalgorithmus aus der Klasse der Single-Slice Rebinning Algorithmen, das Advanced Single-Slice Rebinning (ASSR), zur Verfügung, der alle Erwartungen der klinischen CT zu erfüllen scheint [M. Kachelrieß, S. Schaller, and W.A. Kalender. Advanced single-slice rebinning in cone-beam spiral CT. Med. Phys. 27(4):754-772, April 2000][H. Bruder, M. Kachelrieß, S. Schaller, and T. Mertelmeier. Performance of approximate cone-beam reconstruction in multislice computed tomography. SPIE Medical Imaging Conference Proc. 3979:541-555, 2000][H. Bruder, M. Kachelrieß, S. Schaller, K. Stierstorfer, and T. Flohr. Single-slice rebinning reconstruction in spiral cone-beam computed tomography. IEEE Transactions on Medical Imaging 19(9): 873-887, September 2000]. ASSR sortiert die gemessenen Rohdaten in zweidimensionale Sinogramme — dies ist die matrixförmige Darstellung der Werte aller möglichen Linienintegrale durch eine Ebene — auf gekippten Ebenen um. Diese werden dann mit einer 2D Rekonstruktion (z.B. gefilterter Rückprojektion) rekonstruiert. Die entstehenden gekippten Bilder müssen anschließend in ein reguläres Volumen uminterpoliert werden (Bild-zu-Volumen Interpolation).

Um der Anforderung nach beliebigem Pitch — das ist das Verhältnis zwischen dem Tischvorschub pro Rotation und der z-Ausdehnung des Strahlenkegels — nachzukommen, müssen gekippte Bilder unterschiedlichen Approximationsgrädes, d.h. unterschiedlicher Bildqualität, berechnet werden. Diese Primärbilder tragen üblicherweise mit gleichen Gewichten zum Volumen bei. Durch die Verwendung nicht-optimaler Bilder ist die volle Dosisnutzung (bzw. Detektornutzung) bei beliebigem Pitch möglich. Allerdings ist die so erreichte Bildqualität schlechter als bei alleiniger Verwendung der optimalen Ebenen [S. Schaller, K. Stierstorfer, H. Bruder, M. Kachelrieß, and T. Flohr. Novel approximate approach for high-quality image reconstruction in helical cone beam CT at arbitrary pitch. SPIE Medical Imaging Conference Proc., 2001]. Der Anwender trifft also bereits bei der Wahl der Scanparameter eine Entscheidung über die Bildgüte und kann retrospektiv (nach dem Scan) keine Verbesserung erreichen.

Obwohl die Erweiterung von ASSR auf beliebige Pitchwerte eine wesentliche Anforderung der medizinischen CT erfüllt, können andere, ebenso wichtige Erfordernisse nicht erfüllt werden. Dies sind zum Beispiel Rekonstruktionsverfahren zur herzphasenkorrelierten Rekonstruktion, die in den letzten Jahren für MSCT entwickelt wurden [M. Kachelrieß, W.A. Kalender. ECG-correlated image reconstruction from subsecond spiral CT scans of the heart. Med. Phys. 25(12): 2417-2431, December 1998][M. Kachelrieß, S. Ulzheimer, and W.A. Kalender. ECG-correlated image reconstruction from subsecond multi-slice spiral CT scans of the heart. Med. Phys. 27(8):1881-1902, August 2000][M. Kachelrieß, S. Ulzheimer, and W.A. Kalender. ECG-correlated imaging of the heart with subsecond multislice CT. IEEE Transactions on Medical Imaging 19(9):888-901, September 2000] und sich inzwischen als Standard in der medizinischen CT etablieren konnten. Diese Verfahren erlauben eine 4D Rekonstruktion der gemessenen Daten unter der Voraussetzung, dass die Objektbewegung periodisch ist und die Datenaufnahme überlappend ist (kleiner Pitch). Sie lassen sich auf andere periodische Bewegungen, z.B. Patientenatmung, generalisieren. Es existieren noch keine Ideen, wie die phasenkorrelierte Bildgebung von den zweidimensionalen MSCT-Daten auf dreidimensionale CBCT-Daten zu erweitern ist. Der einfachste Ansatz, die Kegelstrahlgeometrie einfach zu ignorieren und die zweidimensionalen MSCT phasenkorrelierten Algorithmen zu verwenden, ist mit dem Nachteil verbunden, dass mit der Anzahl der simultan gemessenen Schichten zunehmende Bildartefakte akzeptiert werden müssen.

Es erscheint deshalb wünschenswert, den CBCT Algorithmus ASSR oder verwandte Ansätze auf die Möglichkeit zu erweitern, retrospektiv die Qualität, die Herzphase oder andere Merkmale des rekonstruierten Volumens, möglichst in Echtzeit, zu verändern.

Zusammenfassend lassen sich folgende Ziele festhalten:

1. Ein Spiral-Computertomograph, Mehrschicht-Spiral-Computertomograph oder Kegelstrahl-Spiral-Computertomograph, der eine flexible Bild-zu-Volumen (Image-to-Volume, I2V) Interpolation der gekippten Ebenen, falls erwünscht auch in Echtzeit, erlaubt.
($\rightarrow$Patentanspruch 1, 2, 3, 4)
2. Eine Methode, die retrospektiv (nach dem Scan) erlaubt, den Approximationsgrad der Rekonstruktion und somit die Bildgüte zu wählen.
($\rightarrow$Patentanspruch 5, 6)
3. Eine Möglichkeit, phasengewichtete Volumina zu erstellen.
($\rightarrow$Patentanspruch 7, 8)

**Lösung**

Der in Patentanspruch 1 angegebenen Erfindung liegt die Aufgabe zugrunde, für einen Spiral-Computertomographen, Mehrschicht-Spiral-Computertomographen oder Kegelstrahl-Spiral-Computertomographen mit Rekonstruktion von beliebig orientierten und/oder gekrümmten Ebenen (ASSR, damit verwandte Algorithmen oder andere Algorithmen die Daten auf einer Untermannigfaltigkeit zur Verfügung stellen) ein merkmalsbasiertes Gewichtungsverfahren zu entwickeln.

Die Aufgabe ist erfindungsgemäß gelöst durch die Merkmale des Patentanspruchs.

Die Erfindung ist nachfolgend erläutert. Dabei werden als Beispiele jeweils auf die Möglichkeit mit variablen Pitchwerten zu scannen, als auch auf das Herz Bezug genommen, ohne dass damit die Allgemeingültigkeit der Aussagen eingeschränkt werden soll. Insbesondere ist auch die MSCT (Anzahl M der simultan gemessenen Schichten typischerweise 1 bis 4) miteinbezogen.

Die oben genannten Ziele lassen sich im Wesentlichen dadurch erreichen, dass man bei der Bild-zu-Volumen Interpolation den einzelnen gekippten Primärbildern unterschiedliche Gewichte zuordnet. Bei dem bisherigen Verfahren werden keine Unterschiede zwischen den einzelnen Bildern gemacht und alle Ebenen mit gleichem Gewicht in das Volumen eingetragen [M. Kachelrieß, S. Schaller, and W.A. Kalender. Advanced single-slice rebinning in cone-beam spiral CT. Med. Phys. 27(4):754-772, April 2000][S. Schaller, K. Stierstorfer, H. Bruder, M. Kachelrieß, and T. Flohr. Novel approximate approach for high-quality image reconstruction in helical cone beam CT at arbitrary pitch. SPIE Medical Imaging Conference Proc., 2001]. Formuliert man die Rekonstruktionsebene $n$ = 1,...,$N$ als $z_n(x,y)$, was implizit die Tatsache widerspiegelt, dass die Ebenen nur wenig aus der x-y-Ebene herausgekippt sind, und bezeichnet man mit $f_n(x,y)$ den Wert des rekonstruierten Objekts am Punkt $(x,y,z_n(x,y))$, dann lässt sich die bisherige Bild-zu-Volumen Interpolation darstellen als:

$$f(x,y,z) = \frac{\sum_n \int dx'\, dy'\, dz'\, \delta(z_n(x',y') - z')\, k(x',y',z',x,y,z)\, f_n(x',y')}{\sum_n \int dx'\, dy'\, dz'\, \delta(z_n(x',y') - z')\, k(x',y',z',x,y,z)}$$

Dabei wird mit $f(x,y,z)$ das resultierende Volumen bezeichnet, die Dirac'sche Deltafunktion $\delta(\cdot)$ sorgt dafür, dass die Integrale sich über die ensprechende Ebene erstrecken und der (ortsabhängige) Faltungskern $k(x',y',z',x,y,z)$ steuert die Interpolation in den drei Dimensionen. Der Nenner der Gleichung ist zur korrekten Normierung nötig; der Faltungskern k muss stets so gewählt sein, dass dieser Normierungsfaktor an allen Punkten $(x,y,z)$ positiv ist.

Die oben geforderte Gewichtung erfordert die Zuweisung einer orts- und merkmalsabhängigen Gewichtsfunktion $w_{n,\alpha}(x,y)$ zur jeweiligen Rekonstruktionsebene; die Bild-zu-Volumen Interpolation erweitert sich damit auf folgende Operation:

$$f_\alpha(x,y,z) = \frac{\sum_n \int dx'\, dy'\, dz'\, \delta(z_n(x',y') - z')\, k(x',y',z',x,y,z)\, w_{n,\alpha}(x',y')\, f_n(x',y')}{\sum_n \int dx'\, dy'\, dz'\, \delta(z_n(x',y') - z')\, k(x',y',z',x,y,z)\, w_{n,\alpha}(x',y')}\ .$$

Der Index $\alpha$ steht dabei für einen oder mehrere Zusatzparameter, die während der Bild-zu-Volumen Interpolation gewählt oder auch vom Anwender frei manipuliert werden können. Da sich obige Gleichung im Allgemeinen auf wenige Rechenoperationen bei der Darstellung einer oder nur weniger Schichten von $f_\alpha(x,y,z)$, z.B. bei Darstellung von $f_\alpha(x,y,z_R)$ wobei mit $z_R$ die darzustellende z-Position bezeichnet ist, beschränkt, lässt sich diese Berechnung in Echtzeit durchführen um dem Anwender das Resultat unmittelbar zu zeigen.

O.B.d.A. wurde hier die Darstellung $z_n(x,y)$ gewählt um übersichtliche Gleichungen zu erhalten. Das vorgeschlagene Verfahren kann aber auf beliebige Untermannigfaltigkeiten des dreidimensionalen oder eines mehrdimensionalen Raumes angewendet werden. Dies beinhaltet insbesondere die Möglichkeit gekrümmte Primärebenen (bei ASSR handelt es sich hingegen um flache Primärebenen) in ein reguläres Volumen umzuinterpolieren.

Mit dem Gewichtungsverfahren lässt sich das Problem der nicht-optimalen Ebenen lösen. Um ein Volumen zu generieren, zu dem trotz niedrigen Pitch nur die optimalen Ebenen beitragen, müssen für weniger optimale Ebenen niedrige Gewichte gewählt werden. Als Maß für den Approximationsgrad kann zum Beispiel die geometrische Abweichung $\Delta_{Geom,n}(x,y)$ der gemessenen Strahlen zur Rekonstruktionsebene dienen. Diese kann im einfachsten Fall ortsunabhängig gewählt werden; in Referenz [M. Kachelrieß, S. Schaller, and W.A. Kalender. Advanced single-slice rebinning in cone-beam spiral CT. Med. Phys. 27(4):754-772, April 2000] wird als Maß des Approximationsgrades die mittlere quadratische Abweichung der Spiraltrajektorie zur Rekonstruktionsebene $\Delta_{mean}$ vorgeschlagen. Eine mögliche Lösung besteht in der Wahl

$$w_{n,q}(x,y) = \left( \frac{1}{\Delta_{\mathrm{Geom},n}(x,y)} \right)^q$$

wobei der Qualitätsparameter $q$ vom Anwender verwendet werden kann um zwischen niedrigem Artefaktgehalt und niedrigem Rauschen abzuwägen.

Werden nur die Ebenen mit bester Qualität selektiert ($q = \infty$), so resultiert eine geringere Detektornutzung, d.h. höheres Bildpunktrauschen und schlechtere Dosiseffizienz, bei dafür verbessertem Artefaktverhalten.

Auf ähnliche Art und Weise kann eine phasenselektive Rekonstruktion durchgeführt werden. Statt eine Gewichtung mit Hilfe der geometrischen Abweichung durchzuführen, kann hier die Phasenabweichung verwendet werden. Bezeichnet man die gewünschte Rekonstruktionsphase mit $c_R$ (vgl. [M. Kachelrieß, W.A. Kalender. ECG-correlated image reconstruction from subsecond spiral CT scans of the heart. Med. Phys. 25(12): 2417-2431, December 1998][M. Kachelrieß, S. Ulzheimer, and W.A. Kalender. ECG-correlated image reconstruction from subsecond multi-slice spiral CT scans of the heart. Med. Phys. 27(8):1881-1902, August 2000][M. Kachelrieß, S. Ulzheimer, and W.A. Kalender. ECG-correlated imaging of the heart with subsecond multislice CT. IEEE Transactions on Medical Imaging 19(9):888-901, September 2000]) und das zur Ebene $n$ beitragende Phasenintervall mit $[c_n^{\mathrm{Start}}, c_n^{\mathrm{End}}]$ und definiert man z.B. ein mittleres quadratisches Abweichungsmaß wie folgt,

$$\Delta_{\mathrm{Phase},n}^2 = \frac{1}{c_n^{\mathrm{End}} - c_n^{\mathrm{Start}}} \int_{c_n^{\mathrm{Start}}}^{c_n^{\mathrm{End}}} dc\, (c - c_R)^2 \,,$$

so lässt sich damit eine Phasengewichtung der einzelnen Ebenen vornehmen:

$$w_{n,q(x,y)} = \left( \frac{1}{\Delta_{\mathrm{Phase},n}} \right)^{q(x,y)} \,.$$

Bei der Phasenarithmetik wurde implizit eine korrekte Berücksichtigung der Moduloeigenschaften von $c$ angenommen; die Phasenwerte sind also modulo $[0,1]$ zu behandeln. Die Tatsache, dass hier der Qualitätsparameter als Funktion der Ortskoordinate $(x, y)$ vorgeschlagen wird, erlaubt eine gezielte Gewichtung bestimmter Bildbereiche. Allerdings sollen weder die Wahl der Phasenabweichung noch die Wahl der Einzelgewichte die Allgemeinheit einschränken, sondern vielmehr als erklärendes Beispiel verstanden werden. Konkrete Anwendungsmöglichkeiten bestehen insbesondere in der Herzbildgebung (daher der Variablenname $c$ für "cardiac phase") als auch bei atemkorrelierter Rekonstruktion.

Anhand der oben angeführten Beispiele einer Gewichtung gemäß der geometrischen Abweichung und der Phasenabweichung sei hier eine mögliche Kombination der beiden Ansätze demonstriert. Es bezeichne $\Delta_{Geom,n}(x,y)$ den geometrischen Approximationsgrad der Ebene $n$ und es sei $\Delta_{Phase,n}(x,y)$ ein ortsabhängiges Maß der Phasenabweichung der Ebene $n$ von einer gegebenen Zielphase. Die Parameter $q_{Geom}(x,y)$ sowie $q_{Phase}(x,y)$ steuern die Qualität (ortsabhängig) in Bezug sowohl auf geometrische Abbildungsgenauigkeit (räumlich) als auch auf Phasengenauigkeit (zeitlich). Dann kann z.B. mit

$$w_n(x,y) = \left( \frac{1}{\Delta_{Geom,n}(x,y)} \right)^{q_{Geom}(x,y)} \times \left( \frac{1}{\Delta_{Phase,n}(x,y)} \right)^{q_{Phase}(x,y)}$$

das erwünsche Verhalten erzielt werden.

**Patentansprüche**

1. Computertomograph mit Mehrzeilendetektor, der die aus Spiral-CT-Aufnahmedaten, Mehrschicht-Spiral-CT Aufnahmedaten oder Kegelstrahl-Spiral-CT-Aufnahmedaten erzeugten, beliebig orientierten, eventuell gekrümmten Ebenen nach diesen Ebenen zugeordneten Merkmalen gewichtet in ein reguläres Volumen interpoliert.

2. Computertomograph nach Anspruch 1, der bei Mehrfachmerkmalen auch Kombinationen der Gewichte (z.B. Produkt der Einzelgewichte) ermöglicht.

3. Computertomograph nach Anspruch 2, der die Bild-zu-Volumen (Image-to-Volume, I2V) Gewichtung wahlweise in Echtzeit durchführen kann und somit dem Anwender online Manipulationsmöglichkeiten zur Verfügung stellt.

4. Computertomograph nach Anspruch 3, der den gekippten Ebenen ortsabhängige oder ortsunabhängige Gewichte $w_n(x,y)$ zuordnet.

5. Computertomograph nach Anspruch 4, der diesen Ebenen Gewichte zuordnet, die eine Funktion des ortsabhängigen geometrischen Approximationsgrades der jeweiligen Ebene sind.

6. Computertomograph nach Anspruch 5, der die geometrische Abweichung $\Delta_n(x,y)$ nach der Formel $w_n(x,y) = \Delta_n(x,y)^{-q(x,y)}$ mit einem positiven Qualitätsfaktor $q(x,y)$ gewichtet. Für kleine Werte werden alle Ebenen nahezu gleich gewichtet, für große Werte von $q_n(x,y)$ werden die schlechten Ebenen nur gering gewichtet.

7. Computertomograph nach Anspruch 4, der den gekippten Ebenen Gewichte zuordnet, die eine Funktion des zeitlichen Approximationsgrades sind.

8. Computertomograph nach Anspruch 7, der die zeitliche Abweichung $\Delta_n(x,y)$ nach der Formel $w_n(x,y) = \Delta_n(x,y)^{-q(x,y)}$ mit einem positiven Qualitätsfaktor $q(x,y)$ gewichtet. Für kleine Werte werden alle Ebenen nahezu gleich gewichtet, für große Werte von $q_n(x,y)$ werden die Ebenen mit größerer zeitlicher Abweichung nur gering gewichtet.

European Patent Office

# EUROPEAN SEARCH REPORT

Application Number

EP 01 10 7128

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| D,X | KACHELRIESS M ET AL: "ECG-correlated imaging of the heart with subsecond multislice spiral CT" IEEE TRANSACTIONS ON MEDICAL IMAGING, SEPT. 2000, IEEE, USA, vol. 19, no. 9, pages 888-901, XP002184722 ISSN: 0278-0062 | 1-8 | G06T11/00 |
| Y | * abstract * <br> * page 890 - page 893 * | 3 | |
| X | TAGUCHI K ET AL: "ALGORITHM FOR IMAGE RECONSTRUCTION IN MULTI-SLICE HELICAL CT" MEDICAL PHYSICS, AMERICAN INSTITUTE OF PHYSICS. NEW YORK, US, vol. 25, no. 4, 1 April 1998 (1998-04-01), pages 550-561, XP000782534 ISSN: 0094-2405 * page 554 * | 1-6 | |
| X | EP 0 982 682 A (GEN ELECTRIC) 1 March 2000 (2000-03-01) * abstract * * paragraph '0005! * * paragraph '0036! - paragraph '0039! * | 1-5 | TECHNICAL FIELDS SEARCHED (Int.Cl.7) <br> G06T |
| D,X | SCHALLER S ET AL: "Novel approximate approach for high-quality image reconstruction in helical cone beam CT at arbitrary pitch" MEDICAL IMGING 2001, PROCEEDINGS OF THE SPIE, vol. 4322, February 2001 (2001-02), pages 113-127, XP001037818 * abstract * * page 116 - page 117 * | 1-6 | |

-/--

The present search report has been drawn up for all claims

| Place of search | Date of completion of the search | Examiner |
|---|---|---|
| MUNICH | 5 December 2001 | Meinl, W |

EPO FORM 1503 03.82 (P04C01)

**European Patent Office**

**EUROPEAN SEARCH REPORT**

Application Number

EP 01 10 7128

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| Y | EP 0 919 954 A (PICKER INT INC) 2 June 1999 (1999-06-02) | 3 | |
| A | * abstract * | 1-8 | |
| E | EP 1 113 396 A (GE MED SYS GLOBAL TECH CO LLC) 4 July 2001 (2001-07-04) * abstract * | 1-5 | |

TECHNICAL FIELDS SEARCHED (Int.Cl.7)

The present search report has been drawn up for all claims

| Place of search | Date of completion of the search | Examiner |
|---|---|---|
| MUNICH | 5 December 2001 | Meinl, W |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.** EP 01 10 7128

This annex lists the patent family members relating to the patent documents cited in the above-mentioned European search report.
The members are as contained in the European Patent Office EDP file on
The European Patent Office is in no way liable for these particulars which are merely given for the purpose of information.

05-12-2001

| Patent document cited in search report | | Publication date | Patent family member(s) | | Publication date |
|---|---|---|---|---|---|
| EP 0982682 | A | 01-03-2000 | US<br>EP<br>JP | 6055290 A<br>0982682 A2<br>2000083945 A | 25-04-2000<br>01-03-2000<br>28-03-2000 |
| EP 0919954 | A | 02-06-1999 | US<br>EP<br>JP | 6078639 A<br>0919954 A1<br>11239346 A | 20-06-2000<br>02-06-1999<br>31-08-1999 |
| EP 1113396 | A | 04-07-2001 | US<br>CN<br>EP<br>JP | 6324247 B1<br>1310983 A<br>1113396 A2<br>2001204723 A | 27-11-2001<br>05-09-2001<br>04-07-2001<br>31-07-2001 |

EPO FORM P0459

For more details about this annex : see Official Journal of the European Patent Office, No. 12/82